# EUROPEAN PATENT APPLICATION

(11) **EP 3 210 964 A1**
(43) Date of publication of application: **30.08.2017**
(21) Application number: 17152505.8
(22) Date of filing: 18.07.2006
(51) Int. Cl.: C07C 211/63, C07C 233/40, C07C 327/30, A61K 31/216, A61P 29/00, A61P 43/00

(54) **POSITIVELY CHARGED WATER-SOLUBLE PRODRUGS OF IBUPROFEN WITH VERY FAST SKIN PENETRATION RATE**

(62) Divisional of application: 06780126.6
(71) Applicant: Techfields Biochem Co. Ltd, Shanghai N/A 200444 (CN); Yu, Chongxi, Kensington, MD 20895 (US)
(72) Inventor: YU, Chongxi, Kensington, MD 20895 (US); Xu, Lina, Shanghai, 200444 (CN)
(74) Representative: Finnegan Europe LLP

(57) **Abstract**

Pro-drugs of ibuprofen comprising a thiopropionate or propionamide moiety, wherein the amino group is tertiary and protonated. This positively charged amino groups of these pro-drugs not only largely increases the solubility of the drugs, but also bonds to the negative charge on the phosphate head group of membranes and pushes the pro-drug into the cytosol. Diethylaminoethyl 2-(p-isobutylphenyl) propionate.AcOH for example diffuses through human skin 250 times faster than ibuprofen itself and 125 times faster than ethyl 2-(p-isobutylphenyl) propionate. In plasma, more than 90% of these pro-drugs can change back to the drug in a few minutes. The prodrugs can be used medicinally in treating any ibuprofen-treatable conditions in humans or animals and be administered not only orally, but also transdermally for any kind of medical treatments and avoid most of the side effects of ibuprofen, most notably GI disturbances such as dyspepsia, gastroduodenal bleeding, gastric ulcerations, and gastritis. Controlled transdermal administration systems of the prodrug enables the ibuprofen to reach constantly optimal therapeutic blood levels to increase effectiveness and reduce the side effects of ibuprofen.

## Description

### Technical Field

The present invention relates to the preparations of positively charged and water-soluble pro-drugs of 2-(p-isobutylphenyl) propionic acid (ibuprofen) and their medicinal use in treating any ibuprofen-treatable conditions in humans or animals. More specifically , the present invention is to overcome the side effects that are associated with the use of ibuprofen. These pro-drugs can be administered orally or transdermally.

### Background Art

Ibuprofen is a member of the propionic acid group of nonsteroidal anti-inflammatory drugs. The synthesis of ibuprofen was originally reported in 1964 (J.S. Nicholson and S.S. Adams, Br. Patent No. 971, 700) and first used medicinally in Europe. Ibuprofen is more potent than aspirin in anti-inflammatory and prostaglandin biosynthesis inhibition assays. 'PDR Generics' (PDR Generics, 1996, second edition, Medical Economics, Montvale, New Jersey, pg 243) has listed many medical uses of ibuprofen. Ibuprofen is used for the relief of rheumatoid arthritis and osteoarthritis symptoms, the reduction of fever, and the treatment of dysmenorrhea. Ibuprofen is used alone or as an adjunct in the treatment of Bartter's syndrome and chronic uveitis, both anterior and posterior. It is also used in IUD-associated uterine bleeding, and prophylactically to reduce the severity of nausea and prevent radiation-induced vomiting in patients receiving pelvic irradiation. Ibuprofen is also prescribed for diabetic neuropathy, acute migraine headaches, and is used in hemophilic arthropathy. It is also used in the treatment of bone loss (Jee; Webster S. S. U.S. Pat. No. 5,604,259), prevention or treatment of sunburn (Sunshine: Abraham. U.S. Pat. No. 5,100,918)

Unfortunately, a number of side effects are associated with the use of ibuprofen, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of ibuprofen into the host by formulation. Susan Milosovich, et. al. designed and prepared testosteronyl-4-dimethylaminobutyrate.HCl (TSBH), which has a lipophilic portion and a tertiary amine groups that exists in the protonated form at physiological pH. They found that the prodrug (TSBH) diffuses through human skin -60 times faster than does the drug (TS) itself [Susan Milosovich, et al., J. Pharm. Sci., 82, 227(1993).

### Disclosure of Invention

### Technical Problem

Ibuprofen has been used medicinally for more than 30 years and ibuprofen is more potent than aspirin in anti-inflammatory and prostaglandin biosynthesis inhibition. Ibuprofen is indicated for the relief of rheumatoid arthritis and osteoarthritis symptoms, the relief of mild to moderate pain, the reduction of fever, and the treatment of dysmenorrhea. Ibuprofen is used alone or as an adjunct in the treatment of Bartter's syndrome and chronic uveitis, both anterior and posterior. Ibuprofen is also prescribed for diabetic neuropathy, acute migraine headaches, and is used in hemophilic arthropathy. It is also used in IUD-associated uterine bleeding, and prophylactically to reduce the severity of nausea and prevent radiation-induced vomiting in patients receiving pelvic irradiation.

Unfortunately, a number of side effects are associated with the use of ibuprofen, most notably GI disturbances such as dyspepsia, heartburn, vomiting, gastroduodenal bleeding, gastric ulcerations, and gastritis. Gastroduodenal bleeding induced by ibuprofen is generally painless but can lead to fecal blood loss and may cause a persistent iron deficiency anemia.

### Technical Solution

This invention relates to the preparation of novel positively charged pro-drugs of ibuprofen and their use medicinally. These pro-drugs have the general formula (1) "Structure 1". 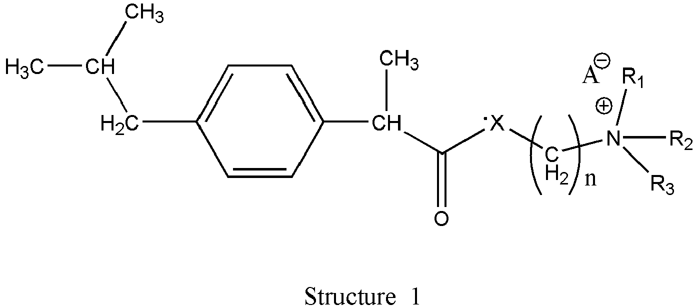 In structure 1, R₁ represents H, one of any alkyl, alkyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S or NH; A⁻ represents Cl⁻, Br⁻ , F⁻, I⁻, AcO⁻, citrate, or any negative ions; and n=0,1,2,3,4,5,6,7,8,9,10...... All R groups may include C, H, O, S, N atoms and may have single, double, and treble bonds. Any CH₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

The goal of this invention is to avoid the side effects of ibuprofen by increasing the solubility of ibuprofen in gastric juice and the penetration rate of ibuprofen through the membrane and skin barrier which will make it administrable transdermally (topical application). These novel pro-drugs of ibuprofen have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of diethylaminoethyl 2-(ρ-isobutylphenyl) propionate. AcOH and 2-(ρ-isobutylphenyl) propionic acid (ibuprofen) in water were >300 mg and 0.05 mg/ml. In many instances, the slowest or rate-limiting step in the sequence is the dissolution of the drug. Ibuprofen has a very low solubility in gastric juice. It stays in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay in GI tract, the prodrugs will not cause gastric mucosal cell damage.

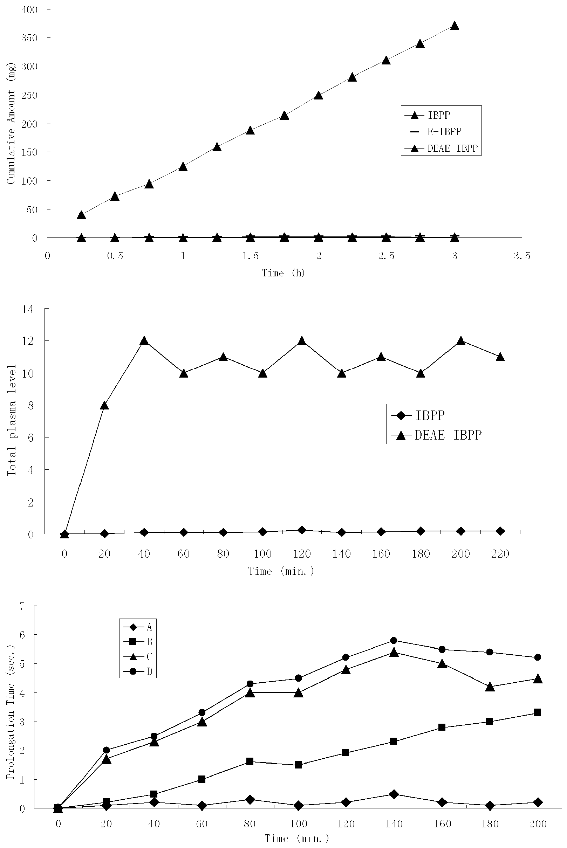

The penetration rates of diethylaminoethyl 2-(ρ-isobutylphenyl) propionate.AcOH and 2-(ρ-isobutylphenyl) propionic acid through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of 2-(ρ-isobutylphenyl) propionic acid (ibuprofen) and diethylaminoethyl 2-(ρ-isobutylphenyl) propionate.AcOH penetrating the skin versus time were determined by a specific high-performance liquid chromatography method. The results using a donor consisting of either a 30% suspension of 2-(ρ-isobutylphenyl) propionic acid (ibuprofen) and ethyl 2-(ρ-isobutylphenyl) propionate and 30% solution of diethylaminoethyl 2-(ρ-isobutylphenyl) propionate.AcOH in 2mL of pH 7.4 phosphate buffer (0.2M) are shown in Figure 1. Apparent flux values of 0.5 mg, 1 mg and 125 mg/cm²/h were calculated for ibuprofen, ethyl 2-(ρ-isobutylphenyl) propionate (the non positive charged normal ester of 2-(ρ-isobutylphenyl) propionic acid), and diethylaminoethyl 2-(ρ-isobutylphenyl) propionate. AcOH. The results suggest that the pro-drug, diethylaminoethyl 2-(ρ-isobutylphenyl) propionate.AcOH diffuses through human skin -250 times faster than does 2-(ρ-isobutylphenyl) propionic acid itself and -125 times faster than does ethyl 2-(ρ-isobutylphenyl) propionate. The normal ester, ethyl 2-(ρ-isobutylphenyl) propionate and 2-(ρ-isobutylphenyl) propionic acid itself have very similar penetration rates (only 2 times difference). The results suggest that the positive charge on the dialkyaminoethyl group has a very important role in the passage of the drug across the membrane and skin barrier. Other prodrugs of the general "Structure 1" have very high penetration rates and are very close to that of diethylaminoethyl 2-(ρ-isobutylphenyl) propionate.AcOH.

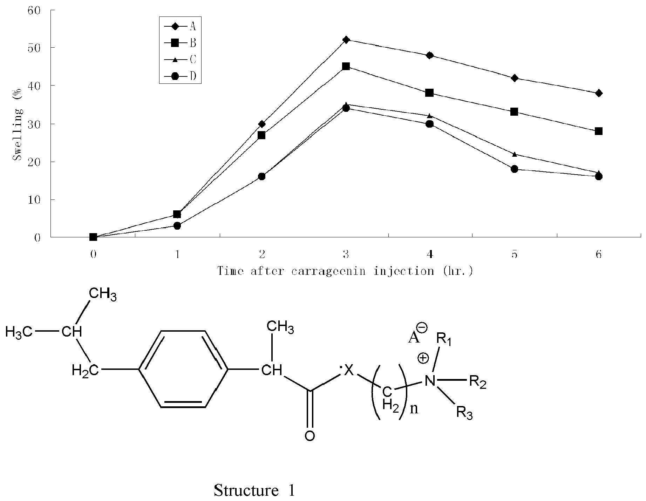

The in vivo rates of penetration of 2-(ρ-isobutylphenyl) propionic acid (IBPP) and diethylaminoethyl 2-(ρ-isobutylphenyl) propionate.AcOH (DEAE-IBPP) through the skin of intact hairless mice were compared. The donor consisted of either a 30% solution of 2-(ρ-isobutylphenyl) propionic acid (IBPP) or diethylaminoethyl 2-(ρ-isobutylphenyl) propionate.AcOH (DEAE-IBPP) in 1 mL of isopropanol applied to a 1 cm² on the backs of the hairless mice. Plasma levels of ibuprofen and diethylaminoethyl 2-(ρ-isobutylphenyl) propionate were determined by a specific high-performance liquid chromatography method. The results (Figure 2) show that the peak levels of diethylaminoethyl 2-(ρ-isobutylphenyl) propionate were reached -30 minutes after application of the donor systems. It takes 1-2 hours for ibuprofen to reach the peak ibuprofen level when it is taken orally. The peaks were -0.2 mg/ml for 2-(ρ-isobutylphenyl) propionic acid (ibuprofen) or -12 mg/ml for diethylaminoethyl 2-(ρ-isobutylphenyl) propionate.AcOH (approximately 60 times difference). -12 mg/ ml of 2-(ρ-isobutylphenyl) propionic acid in plasma is more than 30 times higher than the 2-(ρ-isobutylphenyl) propionic acid plasma level for effective analgesia and effective antiinflammatory activity. This is a very exciting result. It will be very easy and fast to deliver therapeutically effective plasma levels of 2-(ρ-isobutylphenyl) propionic acid into the host by these pro-drugs. These results suggest that the pro-drugs can be administered not only orally, but also transdermally for any kind of medical treatments. The in vivo rates of penetration of other Pro-drugs of the general "Structure 1" are close to that of diethylaminoethyl 2-(ρ-isobutylphenyl) propionate.AcOH.

To check the gastroduodenal bleeding caused by drugs, rats (two groups, each group had 10 rats) were orally administered with 100 mg/kg of ibuprofen or diethylaminoethyl 2-(ρ-isobutylphenyl) propionate.AcOH per day for 21 days. We found an average of 4 mg of fecal blood per gram of feces in the ibuprofen group and none in diethylaminoethyl 2-(ρ-isobutylphenyl) propionate.AcOH group.

Ibuprofen has demonstrated anti-inflammatory, analgesic, antipyretic, and antirheumatic activity. A good prodrug should go back to the drug itself in plasma. Diethylaminoethyl ester group of diethylaminoethyl 2-(ρ-isobutylphenyl) propionate.AcOH can be rapidly cleaved by the enzymes in human plasma in vitro and more than 90% of the pro-drug is changed back to ibuprofen. Due to the pro-drug having a much better absorption rate, the prodrug will have more strength than ibuprofen itself at the same dosage. The analgetic, antipyretic, and anti-inflammatory activities of diethylaminoethyl 2-(ρ-isobutylphenyl) propionate.AcOH were tested using ibuprofen as a comparison. Other compounds of the general 'Structure 1' were tested by the same methods and have very similar results as that of diethylaminoethyl 2-(ρ-isobutylphenyl) propionate.AcOH.

Analgetic activity: The prolongation time of pain the threshold of a mouse tail was determined in accordance with the D'Amour-Smith Method (J. Pharmacol. Exp. Ther., 72, 74(1941)). After 200mg/kg of ibuprofen was administered orally and 200mg/kg of diethylaminoethyl 2-(ρ-isobutylphenyl) propionate.AcOH was administered orally and transdermally, the tails of mice were exposed to heat and the prolongation time of pain threshold was determined. The results obtained are shown in Figure 3. The groups administered 200 mg/kg of diethylaminoethyl 2-(ρ-isobutylphenyl) propionate.AcOH orally (C) and transdermally (D) were shown to exhibit stronger analgetic activity than the group administered 200 mg/kg of ibuprofen.

The number of writhings that occurred when mice were administered an acetic acid solution intraperitoneally were counted, and the rate of inhibition based on the control group was calculated. 42 mice were divided into 7 groups (6 mice each). Ibuprofen (IBPP, 50 mg/kg and 100 mg) was administered to groups B1 and B2 of mice and diethylaminoethyl 2-(ρ-isobutylphenyl) propionate.AcOH (DEAE-IBPP, 50 mg and 100 mg/kg) was administered orally to groups C1 and C2. Diethylaminoethyl 2-(ρ-isobutylphenyl) propionate.AcOH (DEAE-IBPP, 50 mg and 100 mg/kg) was administered transdermally to groups D1 and D2. The A group is the control group. The test compounds were administered to the mice 30 minutes before the acetic acid solution was administered. The results are shown in Table 1.

**Table 1. Te rate of writhings inhibition by and ibuprofen and its pro-drugs**

| Group | A | B1 | B2 | C1 | C2 | D1 | D2 |
|---|---|---|---|---|---|---|---|
| Dose (mg/kg) | 0 | 50 | 100 | 50 | 100 | 50 | 100 |
| No. of writhings | 34.2 | 17.2 | 13.1 | 14.1 | 10.2 | 13.3 | 9.8 |
| % | - | 50 | 62 | 59 | 70 | 61 | 71 |

The results show that diethylaminoethyl 2-(ρ-isobutylphenyl) propionate.AcOH demonstrates better analgetic activity than ibuprofen. Other compounds of the general 'Structure 1' show similar analgetic activity.

Antipyretic activity: Rats received a sterilized E. coli suspension as a pyrogen. 56 rats were divided into 7 groups. The control group is group A. 2 hours later, ibuprofen (IBPP, B1 for 100mg/kg and B2 for 150mg/kg) and diethylaminoethyl 2-(ρ-isobutylphenyl) propionate.AcOH (DEAE-IBPP, C1 for 100mg/kg and C2 for 150 mg) were administered orally and diethylaminoethyl 2-(ρ-isobutylphenyl) propionate.AcOH (DEAE-IBPP, D1 for 100 mg and D2 for 150 mg/kg) was administered transdermally. The body temperatures of the rats were taken at 90 min. intervals before and after the administration of the test compounds. The results are shown in Table 2.

**Table 2. Antipyretic activity of ibuprofen and its pro-drugs**

| Compound | t=0 min. | t=90 min. | t=180 min. | t=270 min. |
|---|---|---|---|---|
| Control group(A) | 37.35±0.05 | 37.25±0.07 | 37.33±0.05 | 37.32±0.08 |
| IBPP (100mg/kg, B1) | 37.25±0.06 | 36.83±0.05 | 36.80±0.08 | 36.78±0.07 |
| IBPP (150mg/kg, B2) | 37.35±0.09 | 36.59±0.07 | 36.53±0.06 | 36.55±0.05 |
| DEAE-IBPP (100mg/kg, C1, orally) | 37.22±0.07 | 36.40±0.06 | 36.50±0.05 | 36.45±0.08 |
| DEAE-IBPP (150mg/kg, C2, orally) | 37.24±0.08 | 36.30±0.05 | 36.35±0.07 | 36.38±0.08 |
| DEAE-IBPP (100mg/kg, D1, transdermally) | 37.27±0.06 | 36.30±0.06 | 36.35±0.08 | 36.31±0.07 |
| DEAE-IBPP (150mg/kg, D2, transdermally) | 37.26±0.05 | 36.25±0.05 | 36.30±0.07 | 36.20±0.05 |

The results shown that diethylaminoethyl 2-(p-isobutylphenyl) propionate.AcOH demonstrated antipyretic activity at 100 mg/kg dose and better than ibuprofen. The results show that transdermal administration of diethylaminoethyl 2-(p-isobutylphenyl) propionate.AcOH is better than oral administration. Other compounds of the general "Structure 1" show similar antipyretic activity.

Anti-inflammatory activity: 50 mg/kg of diethylaminoethyl 2-(ρ-isobutylphenyl) propionate.AcOH was administered orally or transdermally to rats and 50 mg/kg of ibuprofen was administered orally. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling (%). The results obtained are shown in Figure 4. The results show that diethylaminoethyl 2-(p-isobutylphenyl) propionate.AcOH by oral administration and transdermal administration demonstrated better Anti-inflammatory activity than that of ibuprofen at 50mg/kg by oral administration. Other compounds of the general 'Structure 1' show similar anti-inflammatory activity.

It is also known that a high dose of oral ibuprofen shows an anti-reactive-antiasthmatic activity by inhibition of the cyclooxygenase activity. Due to their very high membrane penetration rate, these prodrugs can be used in treating asthma by spraying into the mouth or nose of the host. They can also be used to treat acne due to their anti-inflammatory properties.

The compounds of the general formula (1) "Structure 1" indicated above can be prepared from ibuprofen or from functional derivatives of ibuprofen, for example, acid halides or mixed anhydrides of the general formula (2) 'Structure 2'. 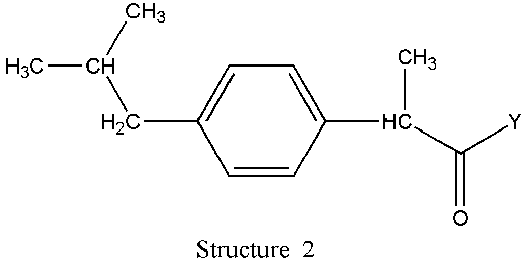 In structure 2, Y represents halogen, alkoxycarbonyl or substituted aryloxy-carbonyloxy, by reaction with compounds of the general formula (3) "Structure 3". 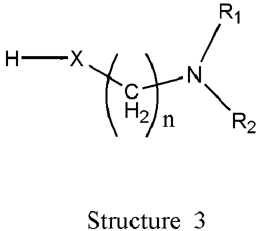 In structure 3, R₁ represents H, one of any alkyl, alkyloxy, alkenyl, or alkynyl residues having 1 to 12 carbon atoms, or aryl residues; R₂ represents H, one of any alkyl, alkyloxy, alkenyl or alkynyl residues having 1 to 12 carbon atoms, or aryl residues; X represents O, S or NH; and n=0,1,2,3,4,5,6,7,8,9,10 .......

The compounds of the general formula (1) "Structure 1" indicated above can be prepared from ibuprofen, by reaction with compounds of the general formula (3) "Structure 3" by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al.

When X represents O, the compounds of the general formula (1) "Structure 1" indicated above can be prepared from metal salts or organic base salts of ibuprofen, by reaction with compounds of the general formula (4) 'Structure 4'. 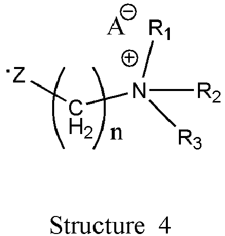 In structure 4, R₁ represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; Z represents halogen, or p-toluenesulphonyl, A⁻ represents Cl⁻, Br⁻, F, I⁻, AcO⁻, citrate, or any negative ions; and n=0,1,2,3,4,5,6,7,8,9,10.......

When X represents O, the compounds of the general formula (1) 'Structure 1' indicated above can be prepared from immobilized base salts of ibuprofen of the general formula (5) "Structure 5". 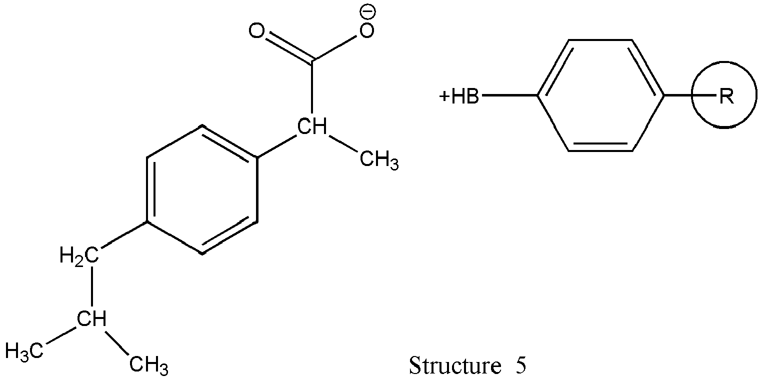 In structure 5, R represents cross-linked resin; B represents any base groups, such as pyridine, piperidine, triethylamine, or other base groups, by reaction with compounds of the general formula (4) "Structure 4".

The present invention relates to pharmaceutical preparations comprising of prodrugs of ibuprofen of the general "Structure 1" in addition to customary auxiliaries and excipients, e.g. in the form of tablets, capsules or solutions for administration orally and in the form of solutions, lotion, ointment, emulsion or gel for administration transdermally. The new active compounds of the general "Structure 1" can be combined with vitamins such as A, B, C or E or beta-carotene, or other pharmaceuticals, such as folic acid, etc. for treating any ibuprofen-treatable conditions in humans or animals.

It is also known that ibuprofen shows an anti-reactive-antiasthmatic activity by inhibition of the cyclooxygenase activity. Due to their very high membrane penetration rate, these pro-drugs can be used in treating asthma by spraying into the mouth or nose of the host.

They can also be used to treat acne and other skin disorders due to their anti-inflammatory properties. They can be used for the treatment and prevention of endothelia dysfunction as well.

These pro-drugs are water-soluble neutral salt and can be tolerated very well by the eye. They can be used for treating eye inflammatory diseases, for treating of ocular pain after corneal surgery, for treating glaucoma or for treating ear inflammatory and/ or painful conditions (otitis).

Transdermal therapeutic application systems of compounds of the general "Structure 1" or a composition comprising at least one compound of the general "Structure 1", as an active ingredient, can be used for treating any ibuprofen-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables ibuprofen to reach constantly optimal therapeutic blood levels to increase effectiveness and reduce the side effects of ibuprofen. These systems can be worn on the wrist, ankle, arm, leg, or any part of body.

### Advantageous Effects

These pro-drugs of ibuprofen have a lipophilic portion and a hydrophilic portion (the amine groups that exist in the protonated form at physiological pH). The positively charged amino groups of these pro-drugs have two major advantages. First, it largely increases the solubility of the drugs; when these new pro-drugs are administered orally in a dosage form such as a tablet, capsule, solution, or suspension, they will dissolve in gastric juice immediately. Second, the positive charge on the amino group of these prodrugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drug was changed back to the drug itself. The pro-drugs have a much better absorption rate, and thus the pro-drugs will have better strength than the ibuprofen itself at the same dosage. The experiment results suggest that the pro-drug, diethylaminoethyl 2-(ρ-isobutylphenyl) propionate.AcOH, diffuses through human skin -250 times faster than ibuprofen itself and -125 times faster than ethyl 2-(ρ-isobutylphenyl) propionate. The in vivo rates of penetration of diethylaminoethyl 2-(ρ-isobutylphenyl) propionate.AcOH through the skin of intact hairless mice were very high. It takes 1-2 hours for ibuprofen tablets to reach the peak ibuprofen plasma level when it is taken orally, but diethylaminoethyl 2-(ρ-isobutylphenyl) propionate.AcOH only took about 30 minutes to reach the peak ibuprofen plasma level. The most exciting result is that the pro-drugs can be administered not only orally, but also transdermally for any type of medical treatment and should avoid most of the side effects of ibuprofen, most notably GI disturbances such as dyspepsia, gastroduodenal bleeding, gastric ulcerations, and gastritis. Another great benefit of transdermal administration of these pro-drugs is that administering medication, especially to children, will be much easier.

### Description of Drawings

Figure 1: Cumulative amounts of 2-(p-isobutylphenyl) propionic acid (IBPP), ethyl 2- (p-isobutylphenyl) propionate (E-IBPP), and diethylaminoethyl 2-(p-isobutylphenyl) propionate. AcOH (DEAE-IBPP) crossing isolated human skin tissue in Franz cells (n=5). IBPP and E-IBPP were applied as a 30% suspension. DEAE-IBPP was applied as 30% solution. In each case, the vehicle was pH 7.4 phosphate buffer (0.2 M).
Figure 2: Total plasma levels of 2-(p-isobutylphenyl) propionic acid (IBPP) after topical application of 1 ml of a 30% solution of 2-(ρ-isobutylphenyl) propionic acid (IBPP) or diethylaminoethyl 2-(ρ-isobutylphenyl) propionate.AcOH (DEAE-IBPP) to the backs of hairless mice (n=5).
Figure 3: The prolongation time of the pain threshold of mice tails after 200 mg/kg of ibuprofen (B) was administered orally, 200 mg/kg of diethylaminoethyl 2-(ρ-isobutylphenyl) propionate.AcOH was administered orally (C) and transdermally (D). A is the control group.
Figure 4. The rate of swelling (%) after a carrageenin injection. 1 hour before the carrageenin injection, 50 mg/kg of ibuprofen was administered orally (B), 50 mg/kg of diethylaminoethyl 2-(ρ-isobutylphenyl) propionate.AcOH was administered orally (C), and transdermally (D). A is the control group.

Structure 1 : In which, R represents H, one of any alkyl, alkyloxy, alkenyl, or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl, or alkynyl residues having 1 to 12 carbon atoms, or aryl residues; X represents O, S or NH; A⁻ represents Cl⁻, Br⁻ , F⁻, I⁻, AcO⁻, acetylsalicylate, citrate, or any negative ions; and n=0,1,2,3,4,5,6,7,8,9,10....... All R groups may include C, H, O, S, N atoms and may have single, double, and treble bonds. Any CH₂ groups may be replaced with O, S, or NH.

### Best Mode

### Preparation of diethylaminoethyl 2-(p-isobutylphenyl) propionate.AcOH

22.5 g (0.1 mol) of 2-(ρ-isobutylphenyl) propionyl chloride was dissolved in 100 ml of chloroform. The mixture was cooled to 0°C. 15 ml of triethylamine and 11.7 g of diethylaminoethanol were added into the reaction mixture. The mixture is stirred for 3 hours at RT. The solid side product was removed by filtration and washed with chloroform (3 x 30 ml). 6 g of acetic acid is added into the chloroform solution with stirring. The organic solution was evaporated off. After drying, it yielded 35 g of the desired product (92%). Hygroscopic product; Solubility in water: 400 mg/ml; Elementary analysis: C₂₁H₃₅NO₄; MW: 365.51. Calculated % C: 69.01; H: 9.65; N: 3.83; O: 17.51; Found % C: 68.98; H: 9.68; N: 3.82; O: 17.52. ¹H-NMR (400 MHz, CDCl₃): δ: 1.10 (d, 6H), 1.52 (d, 3H), 1.56 (t, 6H), 2.21 (s, 3H), 2.22 (m, 1H); 2.51 (d, 2H), 3.28 (m, 4H), 3.52 (m, 2H), 3.78 (m, 1H), 4.52 (t, 2H), 6.8 (b, 1H), 7.06 (d, 2H), 7.07 (d, 2H).

### Mode for Invention

### Preparation of dimethylaminoethyl 2-(p-isobutylphenyl) propionate.AcOH

22.5 g (0.1 mol) of 2-(ρ-isobutylphenyl) propionyl chloride was dissolved in 100 ml of chloroform. The mixture was cooled to 0°C. 15 ml of triethylamine and 8.9 g of dimethylaminoethanol were added into the reaction mixture. The mixture is stirred for 3 hours at RT. 6 g of acetic acid is added into the reaction mixture with stirring. The solid side product was removed by filtration and washed with chloroform (3 x 30 ml). The organic solution was evaporated off. After drying, it yielded 31 g of the desired product (92%). Hygroscopic product; Solubility in water: 400 mg/ml; Elementary analysis: C₁₉H₃₁NO₄; MW: 337.45. Calculated % C: 67.63; H: 9.26; N: 4.15; O: 18.96; Found % C: 67.60; H: 7.28; N: 4.14; O: 18.98. ¹H-NMR (400 MHz, CDCl₃): δ: 1.01 (d, 6H), 1.52 (d, 3H), 2.21 (s, 3H), 2.22 (m, 1H); 2.51 (d, 2H), 2.90 (s, 6H), 3.52 (m, 2H), 3.78 (m, 1H), 4.52 (t, 2H), 6.8 (b, 1H), 7.06 (d, 2H), 7.07 (d, 2H).

### Preparation of S-dimethylaminoethyl 2-(p-isobutylphenyl) thiopropionate.AcOH

22.5 g (0.1 mol) of 2-(ρ-isobutylphenyl) propionyl chloride was dissolved in 100 ml of chloroform. The mixture was cooled to 0°C. 15 ml of triethylamine and 9.3 g of dimethylaminoethyl mercaptan were added into the reaction mixture. The mixture was stirred for 3 hours at RT. 6 g of acetic acid was added into the reaction mixture with stirring. The solid side product was removed by filtration and washed with chloroform (3 x 30 ml). The organic solution was evaporated off. After drying, it yielded 32 g of the desired product (90.5 %). Hygroscopic product; Solubility in water: 300 mg/ml; Elementary analysis: C₁₉H₃₁NO₃S; MW: 353.52. Calculated % C: 64.55; H: 8.84; N: 3.96; O: 13.58, S: 9.07; Found % C: 64.52; H: 8.86; N: 3.95; O: 13.62; S: 9.05.¹ H-NMR (400 MHz, CDCl₃): δ: 1.01 (d, 6H), 1.52 (d, 3H), 2.20 (s, 3H), 2.22 (m, 1H); 2.50 (d, 2H), 2.90 (s, 6H), 3.31 (t, 2H), 3.81 (t, 1H), 3.91 (t, 2H), 6.8 (b, 1H), 7.06 (d, 2H), 7.07 (d, 2H).

### Preparation of N-dimethylaminoethyl 2-(p-isobutylphenyl) propionamide.AcOH

22.5 g (0.1 mol) of 2-(ρ-isobutylphenyl) propionyl chloride was dissolved in 100 ml of chloroform. The mixture was cooled to 0°C. 15 ml of triethylamine and 8.9 g of dimethylaminoethylamine were added into the reaction mixture. The mixture was stirred for 3 hours at RT. 6 g of acetic acid was added into the reaction mixture with stirring. The solid side product was removed by filtration and washed with chloroform (3 x 30 ml). The organic solution was evaporated off. After drying, yielded 30 g of the desired product (89.1 %). Hygroscopic product; Solubility in water: 300 mg/ml; Elementary analysis: C₁₉H₃₂N₂O₃; MW: 336.47. Calculated % C: 67.82; H: 9.59; N: 8.33; O: 14.27; Found % C: 67.80; H: 9.61; N: 8.31; O: 14.26. ¹H-NMR (400 MHz, CDCl₃): δ: 1.01 (d, 6H), 1.52 (d, 3H), 2.20 (s, 3H), 2.22 (m, 1H); 2.50 (d, 2H), 2.90 (s, 6H), 3.50(t, 2H), 3.64 (t, 2H), 3.89 (m, 1H), 6.8 (b, 1H), 7.06 (d, 2H), 7.07 (d, 2H), 7.8 (b, 1H).

### Preparation of S-diethylaminoethyl 2-(p-isobutylphenyl) thiopropionate.AcOH

20.6 g (0.1 mol) of 2-(ρ-isobutylphenyl) propionic acid was dissolved in 100 ml of dichloromethane (DCM). The mixture was cooled to 0°C. 20.6 g of 1,3-Dicyclohexylcarbodiimid was added into the reaction mixture. The mixture was stirred for 30 minutes at 0°C. 13.4 g (0.1 mol) of diethylaminoethyl mercaptan was added into the reaction mixture. The mixture was stirred for 3 h at RT. 6 g of acetic acid was added into the reaction mixture with stirring. The solid side product was rem oved by filtration and washed with chloroform (3 x 50 ml). The organic solution was evaporated off. After drying, it yielded 34 g of the desired product (89.1 %). Hygroscopic product; Solubility in water: 300 mg/ml; Elementary analysis: C₂₁H₃₅NO₃S; MW: 381.57. Calculated % C: 66.10; H: 9.25; N: 3.67; O: 12.58, S: 8.40; Found % C: 66.07; H: 9.29; N: 3.66; O: 12.60; S: 8.38. ¹H-NMR (400 MHz, CDCl₃): δ: 1.01 (d, 6H), 1.52 (d, 3H), 1.56 (t, 6H) 2.20 (s, 3H), 2.22 (m, 1H); 2.50 (d, 2H), 3.26 (m, 4H), 3.31 (t, 2H), 3.81 (t, 1H), 3.91 (t, 2H), 6.8 (b, 1H), 7.06 (d, 2H), 7.07 (d, 2H).

### Preparation of N-dimethylaminopropyl 2-(p-isobutylphenyl) propionamide.AcOH

20.6 g (0.1 mol) of 2-(ρ-isobutylphenyl) propionic acid was dissolved in 100 ml of acetonitrile. 32.1 g of O-(Benzotriazol-1-yl)-N,N,N',N'-tetramethyluronium tetrafluoroborate and 30 ml of triethylamine were added into the reaction mixture. 13.1 g of dimethylaminopropylamine was added into the reaction mixture. The mixture was stirred for 3 hours at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 32 g of the desired product (91.2%). Hygroscopic product; Solubility in water: 320 mg/ml; Elementary analysis: C₂₀H₃₄N₂O_{3;} MW: 350.5. Calculated % C: 68.54; H: 9.78; N: 7.99; O: 13.69; Found % C: 68.51; H: 9.80; N: 7.98; O: 13.71. ¹H-NMR (400 MHz, CDCl₃): δ: 1.01 (d, 6H), 1.52 (d, 3H), 1.98 (m, 2H), 2.20 (s, 3H), 2.22 (m, 1H); 2.50 (d, 2H), 2.90 (s, 6H), 3.20(m, 2H), 3.24 (m, 2H), 3.89 (m, 1H), 6.8 (b, 1H), 7.06 (d, 2H), 7.07 (d, 2H), 7.8 (b, 1H).

### Preparation of dipropylaminoethyl 2-(p-isobutylphenyl) propionate.AcOH

22.3 g (0.1 mol) of sodium 2-(ρ-isobutylphenyl) propionate was suspended in 180 ml of chloroform. 28.8 g (0.1 mol) of dipropylaminoethyl bromide.HBr was added into the mixture and the mixture was stirred for 5 hours at RT. 8.2 g (0.1 mol) of sodium acetate was added into the reaction mixture with stirring. The mixture is stirred for 2 hours. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 35 g of the desired product (88.9%). Hygroscopic product; Solubility in water: 300 mg/ml; Elementary analysis: C₂₃H₃₉NO₄; MW: 393.56. Calculated % C: 70.19; H: 9.99; N: 3.56; O: 16.26; Found % C: 70.14; H: 10.03; N: 3.55; O: 16.28. ¹H-NMR (400 MHz, CDCl₃): δ: 0.96 (d, 6H), δ: 1.10 (d, 6H), 1.52 (d, 3H), 1.77 (m, 4H), 2.21 (s, 3H), 2.22 (m, 1H); 2.51 (d, 2H), 3.24 (m, 4H), 3.52 (m, 2H), 3.78 (m, 1H), 4.52 (t, 2H), 6.8 (b, 1H), 7.06 (d, 2H), 7.07 (d, 2H).

### Preparation of dipropylaminoethyl 2-(p-isobutylphenyl) propionate.AcOH

60 g of Polymer-bound triethylamine (3 mmol/g, 100-200 mesh) was suspended in 180 ml of chloroform. 20.6 g (0.1 mol) of 2-(ρ-isobutylphenyl) propionic acid was added into the mixture with stirring. 43 g (0.15mol) of dipropylaminoethyl bromide.HBr was added into the mixture and the mixture was stirred for 5 hours at RT. The polymer was removed by filtration and washed with tetrahydrofuran (3 x 50 ml). 8.2 g (0.1 mol) of sodium acetate was added into the reaction mixture with stirring. The mixture was stirred for 2 h. The solid was removed by filtration and washed with chloroform (3 x 50 ml). The solution was concentrated in vacuo to 100 ml. Then 300 ml of hexane was added into the solution. The solid product was collected by filtration and washed with hexane (3 x 100 ml). After drying, it yielded 36 g of the desired product (91.5%). Hygroscopic product; Solubility in water: 350 mg/ml; Elementary analysis: C₂₃H₃₉NO₄; MW: 393.56. Calculated % C: 70.19; H: 9.99; N: 3.56; O: 16.26; Found % C: 70.14; H: 10.03; N: 3.55; O: 16.28. ¹H-NMR (400 MHz, CDCl₃): δ: 0.96 (d, 6H), δ: 1.10 (d, 6H), 1.52 (d, 3H), 1.77 (m, 4H), 2.21 (s, 3H), 2.22 (m, 1H); 2.51 (d, 2H), 3.24 (m, 4H), 3.52 (m, 2H), 3.78 (m, 1H), 4.52 (t, 2H), 6.8 (b, 1H), 7.06 (d, 2H), 7.07 (d, 2H).

### Industrial Applicability

The pro-drugs of the general formula (1) "Structure 1" are superior to ibuprofen. They may be used medicinally in treating any ibuprofen-treatable conditions in humans or animals. They may be used for the relief of signs and symptoms of rheumatoid arthritis and osteoarthritis, the reduction of fever, and the treatment of dysmenorrhea. They are used alone or as an adjunct in the treatment of Bartter's syndrome and chronic uveitis, both anterior and posterior. It is also used in ILTD-associated uterine bleeding, and prophylactically to reduce the severity of nausea and prevent radiation-induced vomiting in patients receiving pelvic irradiation. They are also prescribed for diabetic neuropathy and acute migraine headaches and are used in hemophilic arthropathy. They are also used in treatment of bone loss, prevention or treatment of sunburn. They may be used in the prevention of cancer. Due to their very high membrane penetration rate, these pro-drugs can be used in treating asthma by inhalation to a host. They can be used to treat acne due to their anti-inflammatory properties. These pro-drugs are water-soluble neutral salt and can be tolerated very well by the eye. They can be used for treating eye inflammatory diseases, for treating of ocular pain after corneal surgery, for treating glaucoma or for treating ear inflammatory and/or painful conditions (otitis).

The present invention therefore provides:
1. The compounds of the general formula (1) "Structure 1" 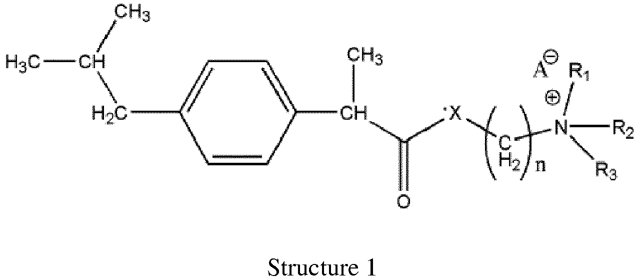 In structure 1, R₁ represents H, one of any alkyl, alkyloxy, alkenyl, or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl, or alkynyl residues having 1 to 12 carbon atoms, or aryl residues; X represents O, S or NH; A represents Cl⁻ , Br , F , I , AcO , acetylsalicylate, citrate, or any negative ions; and n=0,1,2,3,4,5,6,7,8,9,10......... All R groups may include C, H, O, S, N atoms and may have single, double, and treble bonds. Any CH₂ groups may be replaced with O, S, or NH.
2. Process for the preparation of compounds of the general formula (1) "Structure 1" according to 1.
3. Compounds of the general "Structure 1" or a composition comprising of at least one compound of the general formula (1) 'Structure 1', as an active ingredient, according to 1, where they can be administered orally or transdermally, for treating any ibuprofen-treatable conditions in humans or animals. The ibuprofen-treatable conditions include, but are not limited to, pain from a toothache, headache, and arthritis and other inflammatory pain, fever, cancer, dysmenorrhea, Bartter's syndrome and chronic uveitis, both anterior and posterior, IUD-associated uterine bleeding, nausea, radiation-induced vomiting, diabetic neuropathy and acute migraine headache, hemophilic arthropathy, bone loss, and sunburn.
4. Methods for treating any ibuprofen-treatable conditions in humans or animals by administering transdermally to any part of body (in the from of a solution, spray, lotion, ointment, emulsion or gel) to deliver therapeutically effective plasma levels of compounds of the general formula (1) "Structure 1" or a composition comprising of at least one compound of the general formula (1) "Structure 1", as an active ingredient, according to 1.
5. Methods for topically treating pain such as a headache, toothache, and muscle pain, and arthritis and other inflammatory pain in humans or animals by administering to the inflamed area a therapeutically effective amount of the compounds of the general formula (1) "Structure 1" or a composition comprising of at least one compound of the general formula (1) "Structure 1", as an active ingredient, according to 1.
6. Compounds of the general formula (1) "Structure 1" or a composition comprising of at least one compound of the general formula (1) "Structure 1", as an active ingredient, according to 1, may be administered transdermally, for treating acne, sunburn or other skin disorders in the from of a solution, spray, lotion, ointment, emulsion or gel.
7. Compounds of the general "Structure 1" or a composition comprising of at least one compound of the general formula (1) "Structure 1", as an active ingredient, according to 1, are administered by spraying to through the mouth or nose or other parts of body for treating asthma.
8. Compounds of the general "Structure 1" or a composition comprising of at least one compound of the general formula (1) 'Structure 1', as an active ingredient, according to 1, for treating any eye inflammatory diseases, for treating of ocular pain after corneal surgery, for treating glaucoma or for treating ear inflammatory and/or painful conditions (otitis) in humans or animals.
9. Transdermal therapeutic application systems of Compounds of the general formula (1) "Structure 1" or a composition comprising of at least one compound of the general formula(1) "Structure 1", as an active ingredient, according to 1 for treating any ibuprofen-treatable conditions in humans or animals. These systems can be a bandage or a patch comprising of one active substance- containing matrix layer and an impermeable backing layer. The most preferable system is an active substance reservoir, which has a permeable bottom facing the skin. By controlling the rate of release, this system enables the ibuprofen to reach constantly optimal therapeutic blood levels to increase effectiveness and reduce the side effects of ibuprofen.

## Claims

1. A compound chosen from:
S-diethylaminoethyl 2-(p-isobutylphenyl) thiopropionate. HA; and
S-dimethylaminoethyl 2-(p-isobutylphenyl) thiopropionate. HA;
wherein A is a negative ion.

2. A compound or a composition comprising at least one compound as an active ingredient chosen from:
dipropylaminoethyl 2-(p-isobutylphenyl) propionate.HA;
diethylaminoethyl 2-(p-isobutylphenyl) propionate.HA;
N-dimethylaminopropyl 2-(p-isobutylphenyl) propionamide. HA;
S-diethylaminoethyl 2-(p-isobutylphenyl) thiopropionate. HA;
N-dimethylaminoethyl 2-(p-isobutylphenyl) propionamide. HA;
S-dimethylaminoethyl 2-(p-isobutylphenyl) thiopropionate. HA; and
dimethylaminoethyl 2-(p-isobutylphenyl) propionate.HA;
wherein A is a negative ion, for use in the treatment of any ibuprofen-treatable condition
in humans or animals, wherein the compound or composition is administered transdermally, wherein the ibuprofen-treatable condition is selected from the group including
toothache, headache, acute migraine headache, pain of arthritis, inflammatory pain, dysmenorrhea, fever, cancer, Bartter's syndrome, anterior and posterior chronic uveitis, IUD-associated uterine bleeding, nausea, radiation-induced vomiting, diabetic neuropathy, hemophilic arthropathy, bone loss, and sunburn.

3. The compound or composition according to claim 2, wherein the active ingredient is chosen from:
dipropylaminoethyl 2-(p-isobutylphenyl) propionate.AcOH;
diethylaminoethyl 2-(p-isobutylphenyl) propionate.AcOH;
N-dimethylaminopropyl 2-(p-isobutylphenyl) propionamide.AcOH;
S-diethylaminoethyl 2-(p-isobutylphenyl) thiopropionate.AcOH;
N-dimethylaminoethyl 2-(p-isobutylphenyl) propionamide.AcOH;
S-dimethylaminoethyl 2-(p-isobutylphenyl) thiopropionate.AcOH; and
dimethylaminoethyl 2-(p-isobutylphenyl) propionate.AcOH.

4. The compound or composition for use according to claims 2-3, wherein the compound or composition is in the form of a solution, spray, lotion, ointment, emulsion or gel, and administered transdermally to any part of body to deliver therapeutically effective plasma levels.

5. The compound according to claim 1 or a composition comprising at least one compound according to claim 1 as an active ingredient for use in the treatment of pain, wherein the compound or pharmaceutical composition is topically administered to the inflamed area in a therapeutically effective amount.

6. The compound or composition for use according to claims 2 or 5, wherein the pain is headache, toothache, muscle pain, osteoarthritis or other inflammatory pain.

7. The compound according to claims 1-3 or a composition comprising at least one compound according to claims 1-3 as an active ingredient for use in the treatment of acne, sunburn or other skin disorders, and wherein the compound or composition is in the form of a solution, spray, lotion, ointment, emulsion or gel.

8. The compound according to claims 1-3 or a composition comprising at least one compound according to claims 1-3 as an active ingredient for use in the treatment of asthma, wherein the compound or composition is administered by spraying to through the mouth or nose or other parts of body.

9. The compound according to claims 1-3 or a composition comprising at least one compound according to claims 1-3 as an active ingredient for use in the treatment of any eye inflammatory diseases, ocular pain after corneal surgery, glaucoma or ear inflammatory and/or painful conditions in humans or animals.

10. The compound or composition for use according to claim 9, wherein the ear inflammatory and/or painful condition is otitis.

11. **A** transdermal therapeutic application system comprising a compound or a composition comprising at least one compound as an active ingredient chosen from:
dipropylaminoethyl 2-(p-isobutylphenyl) propionate.HA;
diethylaminoethyl 2-(p-isobutylphenyl) propionate.HA;
N-dimethylaminopropyl 2-(p-isobutylphenyl) propionamide. HA;
S-diethylaminoethyl 2-(p-isobutylphenyl) thiopropionate. HA;
N-dimethylaminoethyl 2-(p-isobutylphenyl) propionamide. HA;
S-dimethylaminoethyl 2-(p-isobutylphenyl) thiopropionate. HA; and
dimethylaminoethyl 2-(p-isobutylphenyl) propionate.HA;
wherein A is a negative ion, for use in the treatment of any ibuprofen-treatable condition in humans or animals, wherein the transdermal therapeutic application system enables the ibuprofen to reach constantly optimal therapeutic blood levels to increase effectiveness and reduce the side effects of ibuprofen wherein the ibuprofen-treatable condition is selected from the group including toothache, headache, acute migraine headache, pain of arthritis, inflammatory pain, dysmenorrhea, fever, cancer, Bartter's syndrome, anterior and posterior chronic uveitis, IUD-associated uterine bleeding, nausea, radiation- induced vomiting, diabetic neuropathy, hemophilic arthropathy, bone loss, and sunburn.

12. The transdermal therapeutic application system according to claim 11, wherein the compound is chosen from:
dipropylaminoethyl 2-(p-isobutylphenyl) propionate.AcOH;
diethylaminoethyl 2-(p-isobutylphenyl) propionate.AcOH;
N-dimethylaminopropyl 2-(p-isobutylphenyl) propionamide.AcOH;
S-diethylaminoethyl 2-(p-isobutylphenyl) thiopropionate.AcOH;
N-dimethylaminoethyl 2-(p-isobutylphenyl) propionamide.AcOH;
S-dimethylaminoethyl 2-(p-isobutylphenyl) thiopropionate.AcOH; and
dimethylaminoethyl 2-(p-isobutylphenyl) propionate.AcOH.

13. The transdermal therapeutic application system according to claims 11-12, **characterized in that** the system is a bandage or a patch comprising of an active substance-containing matrix layer and an impermeable backing layer.

14. The transdermal therapeutic application system according to claims 11-13, **characterized in that** the system has an active substance reservoir, which has a permeable bottom facing the skin.

15. The compound according to claim 2, wherein the compound is 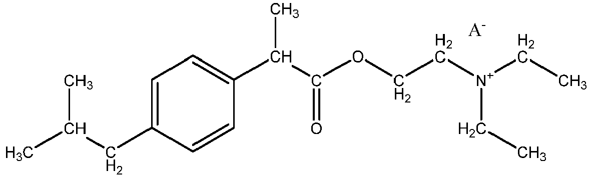 wherein A⁻ is selected from the group consisting of Cl⁻, Br⁻, F⁻, I⁻, AcO⁻, acetylsalicylate and citrate.
